# EUROPEAN PATENT SPECIFICATION

(11) **EP 1 549 230 B1**
(45) Date of publication and mention of the grant of the patent: **17.01.2007**
(21) Application number: 03751015.3
(22) Date of filing: 08.10.2003
(51) Int. Cl.: A61B 17/16

(54) **A REAMER ASSEMBLY**
FRÄSVORRICHTUNG
ENSEMBLE ALESOIR

(30) Priority: 11.10.2002 GB 0223582
(43) Date of publication of application: 06.07.2005
(73) Proprietor: Depuy International Limited, Beeston, Leeds LS11 8DT (GB)
(72) Inventor: Fenton, Gary, Fixby, Huddersfield HD2 2HG (GB)
(74) Representative: Belcher, Simon James
(86) International application number: PCT/GB2003/004303
(87) International publication number: WO 2004/032767

(56) References cited:
- WO-A-00/62718
- FR-A- 995 762
- FR-A- 2 554 709
- US-A- 4 976 740
- US-A- 5 928 238

## Description

The present invention relates to a reamer assembly for preparing the articulating surface of a bone where it articulates in a bone joint for implantation of joint prosthesis component.

It is known to use reamer tools to prepare a bone for implantation of a component of an orthopaedic joint prosthesis. A reamer includes cutting teeth which can be moved against the bone surface to cut the bone. The reamer can be moved reciprocally along an axis to cut the bone, when the cutting teeth are arranged so that they face along that axis. The reamer can be moved rotationally around an axis, in which case the cutting teeth will be arranged so that they face tangentially relative to the axis. In the context of the present invention, a reamer can have cutting teeth which are defined by slits in the wall of a reamer body, with the material of the reamer body deformed out of the plane of the wall at each slit to provide a cutting tooth ("slit-like cutting teeth"). A reamer can have cutting teeth which are provided on the end of the wall of a reamer body, in which case the cutting teeth will be similar to teeth which are provided on a saw.

In a "resurfacing" joint prosthesis, the bone tissue which provides the ball component of a ball and socket joint (for example the humeral component of a shoulder joint or, especially, the femoral component of a hip joint) is prepared to receive a cap-like component which has an outer bearing surface which can be received in the socket component of the joint prosthesis. Preparation involves shaping the bone tissue of the ball component of the natural joint so that its external shape is approximately the same as the cavity within the cap-like prosthesis component. The cavity is rotationally symmetrical, defined by a two or more rotationally symmetrical surfaces. Preparation further involves preparation of a bore in the bone in which a central locating stem of the cap-like component can be located.

US-4976740 discloses a reamer which can be used to remove portions of the head of a femur. The reamer is in the form of a hollow cylindrical shell having four blades depending from its distal edge, which is coaxially aligned with a cannula, having a connector end for engagement with a power drill. The cannula can be fitted into a drilled bore in the femoral head to orientate the shell during the step of cutting the head.

FR-A-2554709 discloses a cutting device for preparing the head of a femur to receive an implant, which comprises a cylindrical central cutter with teeth on its end face to shape the axially facing surface of the head, and a surrounding tubular cutter with teeth on its edge to trim the head peripherally. The device can include a central drill.

The present invention provides a reamer assembly for preparing the articulating surface of a bone, for example to prepare a bone to receive a cap-like component of a resurfacing joint prosthesis, which comprises a reamer guide, and a reamer shell with a sleeve portion which is a sliding fit on the reamer guide.

Accordingly the invention provides a reamer assembly for preparing the articulating surface of a bone where it articulates in a bone joint for implantation or a joint prosthesis component assembly in which the comprises:
a. a reamer guide which can be implanted within the bone to define a reaming axis,
b. a reamer shell for cutting the bone around the reamer guide to a desired shape, including a distal edge which provide cutting teeth in a first array,
the reamer shell comprising a sleeve portion at its proximal end which is a sliding fit on the reamer guide, and having a shell wall which extends proximally from the distal edge with cutting teeth in a second array formed in it as slits which are directed inwardly to cut the external surface of a bone which is located within the shell, the cutting teeth (36) in the second array being radially spaced from the sleeve portion.

The reamer assembly of the present invention has the advantage that the reamer guide can provide enhanced control over the reaming of the bone tissue using the reamer shell, ensuring that the location of the reamed bone tissue is determined accurately relative to a central axis which is defined by the reamer guide. In this way, a sequence of reaming steps which form part of a surgical procedure can be performed on a common axis, for example to create a surface on the bone which includes two or more rotationally symmetrical surfaces. The enhanced control over the creation of multiple rotationally symmetrical surfaces has the advantage that the likelihood of discontinuities in the configuration of the finished bone is reduced. Such discontinuities can interfere with satisfactory performance of the joint prosthesis after implantation, for example by placing unacceptable limits on the range of motion of the joint.

The reamer guide can have cutting surfaces by which it can cut a bore in the bone. The reamer guide can have cutting teeth on its axially extending surfaces. For example, the cutting teeth can be in the form of the cutting surfaces on a drill bit, extending helically along the surface of the guide. Alternatively, they could be in the form of generally axially extending slits. Preferably, the reamer guide has cutting surfaces on its distal end, and a bore extending along its length, so that it can cut and facilitate removal of a core of bone tissue (optionally together with cutting surfaces on its axially extending surfaces). The reamer guide can therefore prepare a bore to receive a central locating stem of a prosthesis component.

Preferably, the reamer guide has one or more depth markings to indicate to the surgeon the depth of penetration of the guide into the bone. The depth markings can be provided by an element which extends from the reamer guide generally transversely relative to the reaming axis.

Preferably, the reamer guide has cutting teeth for cutting the proximal surface of the bone, around the point of entry of the guide into the bone. Preferably, the cutting teeth are provided on the underside of an element which extends from the reamer guide generally transversely relative to the reaming axis. The element will generally be rotationally symmetrical about the reaming axis, for example provided by a circular disk, especially a planar disk. The element can have markings on the side which extends parallel to the reaming axis to indicate the depth of penetration of the element (and therefore the guide as a whole) into the bone.

Preferably, the reamer shell has an internal surface which is configured to engage the element to limit the depth to which the reamer shell can be moved during the step of reaming the bone using the reamer shell. For example, the reamer shell can have a circular recess which an element provided by a circular disk fits into when the shell has reached the extent of its travel.

The reamer shell can include an array of cutting teeth on its distal edge, the wall of the reamer shell extending proximally from the distal edge, preferably substantially parallel to the reaming axis. The reamer shell includes an array of cutting teeth which are formed as slits in the wall of the shell. The reamer shell can include more than one array of cutting teeth, in which the arrays of cutting teeth are designed to create respective rotationally symmetrical bone surfaces. Accordingly, the reamer shell includes an array of cutting teeth on its distal edge, and an array of cutting teeth which are formed as slits in the wall of the shell.

The cutting teeth in the wall of the shell are directed inwardly to cut the external surface of a bone which is located within the shell.

Preferably, the cutting teeth in the wall of the shell are arranged so as to cut a surface of the bone which is inclined to the reaming axis. Preferably, the included angle between the inclined wall of the shell and the reaming axis is at least about 25°, more preferably at least about 35°. Preferably, the included angle between the inclined wall of the shell and the reaming axis is not more than about 70°, more preferably not more than about 55°.

Preferably, at least one of the components of the assembly, especially each of the components of the assembly, has a non-circular portion at its proximal end which can engage a drive unit by which the component can be made to rotate. For example, the end of the component can have a hexagonal cross-section, which can be received in an appropriate hexagonal socket in the head of a powered drive unit. Alternatively, the end of the component might provide a hexagonal socket in which a hexagonal plug on the drive unit can be received.

Components of the reamer assembly can be made from materials which are used conventionally in the manufacture of surgical instruments. Examples of such materials include certain stainless steels. The cutting teeth which are provided on the components of the assembly can be configured in line with existing surgical instruments used in similar applications.

The assembly of the invention can be used to prepare the ball component of a ball and socket joint (for example the humeral component of a shoulder joint or, especially, the femoral component of a hip joint) to receive a cap-like component which has an outer bearing surface which can be received in the socket component of the joint prosthesis. Preparation involves shaping the bone tissue of the ball component of the natural joint so that its external shape is approximately the same as the cavity within the cap-like prosthesis component. The cavity is rotationally symmetrical, defined by a two or more rotationally symmetrical surfaces. For example, one rotationally symmetrical surface can extend generally parallel to the axis of the assembly, which can be cut by cutting teeth on the distal edge of the reamer shell. A rotationally symmetrical surface can be generally inclined to the axis of the assembly, which can be cut by cutting teeth which are formed in the wall of the shell, the wall being arranged at the same angle to the axis of the assembly as is intended for the surface of the bone.

The dimensions of the components of the assembly will depend on the application for which the assembly is intended to be used. For example, when the assembly is used to prepare the head of a femur for implantation of a resurfacing prosthesis component, the diameter of the shell will generally be at least about 15 mm, preferably at least about 20 mm, more preferably at least about 30 mm. The diameter will generally be less than about 60 mm, preferably less than about 50 mm. The length of the part of the reamer guide which extends into the head of the femur will generally be at least about 30 mm, preferably at least about 40 mm, for example at least about 50 mm. The diameter of the reamer guide will generally be less than about 15 mm, for example less than about 10 mm. The diameter will generally be at least about 5 mm. The diameter of the reamer guide can vary along its length; for example, its diameter can increase slightly from the distal tip towards the depth stop.

The sleeve portion of the shell component will be configured so that it is a sliding fit over the reamer guide.

Embodiments of the present invention will now be described by way of example with reference to the accompanying drawings, in which:
Figure 1 is a sectional elevation view of the head of a femur, with the reamer guide component of the reamer of the assembly of the invention implanted in the femur, and the reamer shell component positioned to create rotationally symmetrical external surfaces.
Figure 2 is a side view of the reamer shell component of the assembly of the invention.
Figure 3 is a sectional elevation from one side of the head of a femur and reamer assembly components shown in Figure 1, after the reamer assembly has been driven over the femoral head to create rotationally symmetrical external surfaces.

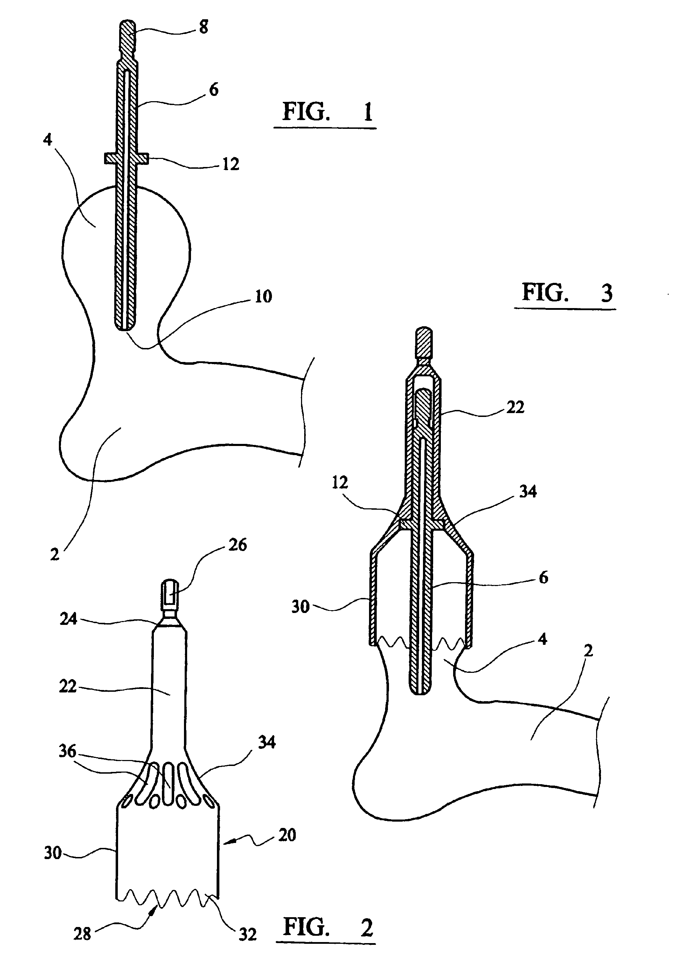

Referring to the drawings, Figure 1 shows the upper portion of a femur 2, including the head 4 which is received in the patient's acetabulum during articulation of the joint. The femur is shown in isolation to show how the present invention can be put into effect to prepare the femur for implantation of a "resurfacing" joint prosthesis component. This can be used to provide a hard wearing bearing surface on the head of the femur, which can articulate with an appropriate prosthesis component which is implanted in the acetabulum. The use of such surgical techniques has the advantage that most of the femoral tissue is preserved.

The preparation of the head of the femur to receive a resurfacing prosthesis component involves the formation of a bore along or at a desired angle to the axis of the head, to receive the stem of the component. The external surface of the head is prepared by the formation of two rotationally symmetrical reamed surfaces. A first surface is aligned parallel with the axis of the head. A second surface extends between the first surface and the axis, the angle between the first and second surfaces being about 135°.

The assembly of the invention includes a reamer guide 6 having a non-circular head 8 which can be engaged by a drive unit, especially a powered rotational drive. The reamer guide is hollow, at least towards its distal end 10, where it is provided at distal end with an array of cutting teeth in the manner of saw teeth. Accordingly, when the reamer guide is directed towards the patient's bone and rotated, the teeth cut a circular bore in the bone, which is received in the bore in the guide.

The reamer guide has a depth indicator 12 which is provided by a planar circular disk, mounted part way along its length. The disk has cutting teeth on its lower surface by which a circular recess can be cut into the bone. Markings on the side of the depth indicate the depth of the recess to the surgeon.

The reamer assembly of the invention includes a reamer shell 20. The reamer shell comprises a sleeve portion 22 towards its proximal end 24 which is configured so that it is a sliding fit over the reamer guide. The shell has a non-circular head 26 which can be engaged by a drive unit, especially a powered rotational drive.

The shell 20 has a hollow cavity 28 within it whose shape corresponds to the intended shape of the femoral head after it has been reamed using the shell. The shell within the cavity is defined in part by a circular first wall 30 which is arranged parallel to the axis of the shell (defined by the sleeve portion 22). The circular wall has an array of cutting teeth 32 in the manner of saw teeth at its end, which is the distal end of the shell. The teeth are directed downwardly so that, when the shell is rotated about the axis of the shell, the teeth can cut a circular shape in a bone positioned in the direction of the axis.

The circular first wall can also have a plurality of slit-like cutting teeth formed in it which are able to remove thin slices of bone tissue from the surface of a bone which is contacted by the first wall when the shell is rotated.

The shell is further defined, in the region between the circular first wall 30 and the sleeve portion 22, by a second wall 34 which is inclined to the axis of the shell defined by the sleeve portion. The angle between the first and second walls is about 135°. As shown in Figure 2, the reamer shell has a plurality of slit-like cutting teeth 36 in the second wall which are able to remove thin slices of bone tissue from the surface of a bone which is contacted by the second wall when the shell is rotated.

As shown in Figure 1, the first step in use of the assembly of the present invention to prepare the femoral head involves creating a bore in the head, aligned with the axis of the head, using a drill. The location and angular orientation of the bore can be determined relative to anatomical features of the bone. It will generally be preferred for the bore to be aligned with the axis of the spherical head of the bone when the reamer is being used to prepare the head of a femur.

The bore which is drilled in the bone is then used to receive a reamer guide 6. The reamer guide is driven into the drilled bore in the patient's bone using an appropriate rotational drive, with the guide cutting into the bone tissue by means of the cutting teeth at the distal end 10 and on the underside of the depth indicator disk 12. The reamer guide is driven into the bone until the disk 12 contacts the surface of the bone, or has penetrated the bone to a desired depth (indicated by the markings on the side of the disk). This results in the formation of a columnar core within the hollow reamer guide, which can be removed from the bone with the reamer guide later in the procedure. The shape and depth of the resulting bore are appropriate having regard to the shape of the stem part of resurfacing implant.

In a second step, the rotational drive is connected to the shell 20 so that it can be driven rotationally. It is positioned over the reamer guide so that the guide is received within the sleeve portion 22 of the shell. The receipt of the reamer guide in the sleeve portion ensures that the shell is aligned with the axis defined by the reamer guide.

Rotation of the shell 20 when in contact with the surface of the patient's bone initially causes a circular cylinder to be cut, by means of the cutting teeth 32 at the distal end of the shell. Continued movement of the shell along the axis defined by the reamer guide causes an inclined surface to be cut by means of the slit-like cutting teeth 36 in the surface of the patient's bone, extending between the cylindrical surface which is cut by the cutting teeth 32 at the distal end of the shell and the axially extending reamer guide. The movement of the shell, to cause the cylindrical and inclined surfaces to be cut in the bone, is limited by the depth indicator disk 12 on the reamer guide, when it is contacted by the internal surface of the shell, as shown in Figure 3.

## Claims

1. A reamer assembly for preparing the articulating surface of a bone (2) where it articulates in a bone joint for implantation of a joint prosthesis component, assembly in which the comprises:
a. a reamer guide (6) which can be implanted within the bone to define a reaming axis,
b. a reamer shell (20) for cutting the bone around the reamer guide to a desired shape, including a distal edge which provide cutting teeth (32) in a first array,
the reamer shell (20) comprising a sleeve portion (22) at its proximal end (24) which is a sliding fit on the reamer guide (6), and having a shell wall (30) which extends proximally from the distal edge with cutting teeth (36) in a second array formed in it as slits (36) which are directed inwardly to cut the external surface of a bone which is located within the shell, the cutting teeth (36) in the second array being radially spaced from the sleeve portion.

2. A reamer assembly as claimed in claim 1, in which the reamer guide (6) has cutting surfaces by which it can cut a bore in the bone.

3. A reamer assembly as claimed in claim 2, in which the cutting surfaces on the reamer guide (6) are on its distal end (10), and in which the guide has a bore extending along its length.

4. A reamer assembly as claimed in claim 1, in which the reamer guide (6) has a depth indicator (12) to indicate the depth of penetration of the guide into the bone.

5. A reamer assembly as claimed in claim 4, in which the depth indicator (12) is provided by an element which extends from the reamer guide (6) generally transversely relative to the reaming axis.

6. A reamer assembly as claimed in claim 4, in which the depth indicator (12) is rotationally symmetrical about the reaming axis.

7. A reamer assembly as claimed in claim 4, in which the reamer shell (20) has an internal surface which is configured to engage the depth indicator to limit the depth to which the reamer shell can be moved during the step of reaming the bone using the reamer shell.

8. A reamer assembly as claimed in claim 1, in which the cutting teeth (36) in the wall of the shell are arranged so as to cut a surface of the bone which is inclined to the reaming axis.

9. A reamer assembly as claimed in claim 8, in which the included angle between the wall of the shell which cuts the inclined surface of the bone and the reaming axis is at least 25°.

10. A reamer assembly as claimed in claim 8, in which the included angle between the wall of the shell which cuts the inclined surface of the bone and the reaming axis is not more than 70°.

11. A reamer assembly as claimed in claim 1, in which the reamer shell includes a third array of cutting teeth which, together with the teeth (36) of the second array, are provided in the shell wall, the cutting teeth of the third array being directed inwardly to cut bone tissue located within the shell, so that the cutting teeth of both the second and third arrays create rotationally symmetrical bone surfaces.

## Patentansprüche

1. Fräseranordnung zum Herstellen der gelenkbildenden Fläche eines Knochens (2), wo er sich in einem Knochengelenk gelenkig bewegt, für das Implantieren einer Gelenkprothesenkomponente, wobei die Anordnung aufweist:
a. eine Fräserführung (6), die innerhalb des Knochens implantiert werden kann, um eine Fräsachse zu definieren,
b. einen Fräsermantel (20) zum Schneiden des Knochens um die Fräserführung in eine gewünschte Form, der eine distale Kante umfaßt, welche Schneidzähne (32) in einer ersten Anordnung bereitstellt,
wobei der Fräsermantel (20) einen Hüllenabschnitt (22) an seinem proximalen Ende (24), welcher im Gleitsitz auf der Fräservornchtung (6) ist aufweist und eine Mantelwand (30) hat, die sich proximal von der distalen Kante mit Schneidzähnen (36) in einer zweiten Anordnung erstreckt, die in ihr als Schlitze (36) ausgebildet sind, die nach innen gerichtet sind, um die Außenfläche eines Knochens zu schneiden, der sich innerhalb der Mantels befindet, wobei die Schneidzähne (36) in der zweiten Anordnung radial von dem Hülsenabschnitt beabstandet sind.

2. Fräseranordnung nach Anspruch 1, bei der die Fräserführung (6) Schneidflächen hat, mit denen sie eine Bohrung in den Knochen schneiden kann.

3. Fräseranordnung nach Anspruch 2, bei der die Schneidflächen auf der Fräserführung (6) sich auf ihrem distalen Ende (10) befinden, und bei der die Führung eine Bohrung hat, die sich über ihre Länge erstreckt.

4. Fräseranordnung nach Anspruch 1, bei der die Fräserführung (6) einen Tiefenindikator (12) hat, um die Tiefe des Eindringens der Führung in den Knochen anzugeben.

5. Fräseranordnung nach Anspruch 4, bei der der Tiefenindikator (12) durch ein Element zur Verfügung gestellt wird, das sich von der Fräserführung (6) im allgemeinen quer zu der Fräsachse erstreckt.

6. Fräseranordnung nach Anspruch 4, bei der der Tiefenindikator (12) rotationssymmetrisch um die Fräsachse ist.

7. Fräseranordnung nach Anspruch 4, bei der der Fräsermantel (20) eine Innenfläche hat, die so ausgelegt ist, daß sie an dem Tiefenindikator anliegt, um die Tiefe zu begrenzen, in die der Fräsermantel während des Schrittes des Fräsens des Knochens unter Verwendung des Fräsermantels bewegt werden kann.

8. Fräseranordnung nach Anspruch 1, bei der die Schneidzähne (36) in der Wand des Mantels so angeordnet sind, daß sie eine Fläche des Knochens schneiden, die zu der Fräsachse geneigt ist.

9. Fräseranordnung nach Anspruch 8, bei der der eingeschlossene Winkel zwischen der Wand des Mantels, die die geneigte Fläche des Knochens schneidet, und der Fräsachse wenigstens 25° ist.

10. Fräseranordnung nach Anspruch 8, bei der der eingeschlossene Winkel zwischen der Wand des Mantels, die die geneigte Fläche des Knochens schneidet, und der Fräsachse nicht mehr als 70° ist.

11. Fräseranordnung nach Anspruch 1, bei der der Fräsermantel eine dritte Anordnung aus Schneidzähnen umfaßt, die zusammen mit den Zähnen (36) der zweiten Anordnung in der Mantelwand vorgesehen sind, wobei die Schneidzähne der dritten Anordnung nach innen gerichtet sind, um Knochengewebe zu schneiden, das sich innerhalb des Mantels befindet, so daß die Schneidzähne sowohl der zweiten als auch der dritten Anordnung rotationssymmetrische Knochenflächen erzeugen.

## Revendications

1. Ensemble alésoir destiné à préparer la surface d'articulation d'un os (2) où il s'articule dans une articulation osseuse pour l'implantation d'un composant de prothèse d'articulation, dans lequel l'ensemble comprend :
a. un guide d'alésoir (6) qui peut être implanté à l'intérieur de l'os pour définir un axe d'alésage,
b. une coquille d'alésoir (20) destinée à couper l'os autour du guide d'alésoir à une forme souhaitée, comprenant un bord distal qui fournit des dents de coupe (32) dans une première rangée,
la coquille d'alésoir (20) comprenant une partie de manchon (22) à son extrémité proximale (24) qui représente un ajustement coulissant sur le guide d'alésoir (6), et ayant une paroi de coquille (30) qui s'étend de manière proximale à partir du bord distal avec des dents de coupe (36) dans une deuxième rangée formée dans celle-ci sous forme de fentes (36) qui sont dirigées vers l'intérieur pour couper la surface externe d'un os qui est situé à l'intérieur de la coquille, les dents de coupe (36) dans la deuxième rangée étant radialement espacées de la partie du manchon.

2. Ensemble alésoir selon la revendication 1, dans lequel le guide d'alésoir (6) a des surfaces de coupe lui permettant de couper un trou dans l'os.

3. Ensemble alésoir selon la revendication 2, dans lequel les surfaces de coupe sur le guide d'alésoir (6) sont sur son extrémité distale (10), et dans lequel le guide a un trou s'étendant sur toute sa longueur.

4. Ensemble alésoir selon la revendication 1, dans lequel le guide d'alésoir (6) a un indicateur de profondeur (12) pour indiquer la profondeur de pénétration du guide dans l'os.

5. Ensemble alésoir selon la revendication 4, dans lequel l'indicateur de profondeur (12) est fourni par un élément qui s'étend à partir du guide d'alésoir (6) de manière globalement transversale par rapport à l'axe d'alésage.

6. Ensemble alésoir selon la revendication 4, dans lequel l'indicateur de profondeur (12) est symétrique en rotation autour de l'axe d'alésage.

7. Ensemble alésoir selon la revendication 4, dans lequel la coquille d'alésoir (20) a une surface interne qui est configurée pour s'engager dans l'indicateur de profondeur afin de limiter la profondeur à laquelle la coquille d'alésoir peut être déplacée pendant l'étape d'alésage de l'as en utilisant la coquille d'alésoir.

8. Ensemble alésoir selon la revendication 1, dans lequel les dents de coupe (36) dans la paroi de la coquille sont agencées de manière à couper une surface de l'os qui est inclinée vers l'axe d'alésage.

9. Ensemble alésoir selon la revendication 8, dans lequel l'angle compris entre la paroi de la coquille qui coupe la surface inclinée de l'os et l'axe d'alésage est d'au moins 25°.

10. Ensemble alésoir selon la revendication 8, dans lequel dans lequel l'angle compris entre la paroi de la coquille qui coupe la surface inclinée de l'os et l'axe d'alésage n'est pas supérieur à 70°.

11. Ensemble alésoir selon la revendication 1, dans lequel la coquille d'alésoir comprend une troisième rangée de dents de coupe qui, conjointement avec les dents (36) de la deuxième rangée, sont prévues dans la paroi de la coquille, les dents de coupe de la troisième rangée étant dirigées vers l'intérieur pour couper le tissu osseux situé à l'intérieur de la coquille, de sorte que les dents de coupe à la fois des deuxième et troisième rangées créent des surfaces d'os symétriques en rotation.
